# Europäisches Patentamt

# European Patent Office

# Office européen des brevets

(11) Veröffentlichungsnummer: **0 050 222**
**B1**

(12) ## EUROPÄISCHE PATENTSCHRIFT

(45) Veröffentlichungstag der Patentschrift:
**21.12.83**

(21) Anmeldenummer: **81107477.2**

(22) Anmeldetag: **21.09.81**

(51) Int. Cl.³: **C 07 C 15/073, C 07 C 2/68**

(54) Verfahren zur kontinuierlichen Herstellung von Ethylbenzol in heterogener Phase.

(30) Priorität: **22.10.80 DE 3039760**

(43) Veröffentlichungstag der Anmeldung:
**28.04.82 Patentblatt 82/17**

(45) Bekanntmachung des Hinweises auf die Patenterteilung:
**21.12.83 Patentblatt 83/51**

(84) Benannte Vertragsstaaten:
**BE DE FR GB IT NL**

(56) Entgegenhaltungen:
**DE - A - 2 659 697**
**DE - A - 2 940 553**
**GB - A - 589 072**
**GB - A - 639 873**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,**
**Carl-Bosch-Strasse 38, D-6700 Ludwigshafen (DE)**

(72) Erfinder: **Heimlich, Guenther, Dr., Nr. 56 1/2,**
**D-8229 Airnring (DE)**
Erfinder: **Tremmel, Gregor, Zeppelinstrasse 12,**
**D-6718 Gruenstadt (DE)**
Erfinder: **Lieb, Manfred, Radestrasse 5,**
**D-6700 Ludwigshafen (DE)**

BUNDESDRUCKEREI BERLIN

## Verfahren zur kontinuierlichen Herstellung von Ethylbenzol in heterogener Phase

Die Erfindung betrifft ein Verfahren zur kontinuierlichen Herstellung von Ethylbenzol in heterogener Phase durch Alkylieren von Benzol mit Ethylen unter Verwendung von Friedel-Crafts-Katalysatoren und unter Auftrennung des Reaktionsgemisches in zwei Phasen, Aufarbeitung der spezifisch leichteren Phase unter Abtrennung des Wertproduktes und Rückführung des Restes der leichten Phase sowie der spezifisch schweren Phase in die Reaktionszone.

Die Umsetzung von Benzol mit Ethylen zu Ethylbenzol in Gegenwart von Friedel-Crafts-Katalysatoren ist ein technisch im großen Umfang ausgeübtes Verfahren. Einzelheiten zu den verschiedenen technisch ausgeführten Verfahren sind in den folgenden Druckschriften beschrieben:

(1)  Kunststoff-Handbuch, Bd. V, Polystyrol, Carl Hauser-Verlag (1969), Seiten 18 bis 23
(2)  DE-AS 1 907 011
(3)  DE-AS 2 445 797
(4)  US-PS 3 751 504
(5)  Ullmann, Enzyklopädie der techn. Chemie Bd. 14, 4. Aufl. 1977, Seiten 673 ff.

Die Herstellung von Ethylbenzol durch Alkylierung von Benzol mit Ethylen in Gegenwart von Friedel-Crafts-Katalysatoren und Promotoren hierfür ergibt in der Regel ein Reaktionsgemisch, das außer dem Wertprodukt Ethylbenzol nicht umgesetztes Benzol sowie Diethylbenzol, Triethylenbenzol und außerdem hochsiedende Polyethylenbenzole enthält. Sowohl das Benzol als auch die mehrfach ethylierten Benzole werden in der Praxis durch Destillation vom Ethylbenzol getrennt und dem Alkylierungsreaktor zugeführt. In Anwesenheit von überschüssigem Benzol entsteht unter Reaktionsbedingungen aus den Polyethylbenzolen durch »Disproportionierung« Ethylbenzol. Auf diese Weise wird eine Ethylbenzolausbeute von ≥ 98%, bezogen auf Benzol, erhalten.

Um Energiekosten niedrig zu halten, wird bei der Ethylbenzolsynthese bei optimalem Umsatz die angewendete Benzolmenge begrenzt. Für das gängige Verfahren, bei dem die Ethylierung und Transethylierung in der gleichen Reaktionszone bei Temperaturen bis 130° C erfolgt, werden maximal 2,0 bis ca. 3 Mol Benzol, bezogen auf 1 Mol Ethylen, angewendet. Wird das Benzol : Ethylen-Verhältnis auf Werte von unter 2,0 verringert, so steigt der Polyethylbenzolgehalt überproportional an, bei gleichzeitiger Abnahme der Benzol- und Ethylbenzolkonzentration im Reaktorauslauf. Hier liegt eine der Begrenzungen, die Ethylbenzolsynthese kostengünstiger durchzuführen.

Eine andere Methode, den Umsatz zu verbessern, besteht darin, die Reaktionstemperatur von 130 auf Werte von ≤ 160° C zu erhöhen. Auch diesem Verfahren sind Grenzen gesetzt. Zwar steigt die Ethylbenzolkonzentration an, aber gleichzeitig dazu auch der Polyethylbenzolgehalt, so daß die Benzolkonzentration sich dementsprechend verringert.

Eine Erhöhung des Umsatzes durch die Anwendung von größeren Mengen an Katalysator bzw. Cokatalysatoren führt nicht zum Ziel.

Es bestand daher die Aufgabe, die vorstehend geschilderten Nachteile bei den bekannten Verfahren zu beseitigen. Die Lösung dieser Aufgabe erfolgt durch die im Patentanspruch gekennzeichneten Maßnahmen.

Das Verfahren zur kontinuierlichen Herstellung von Ethylbenzol in heterogener Phase ist beispielsweise aus (1) bzw. (3) bzw. (5) hinreichend bekannt, so daß auf dieses nachfolgend, soweit die Merkmale des Oberbegriffs des Patentanspruchs betroffen sind, nur kurz eingegangen wird.

Zur Durchführung des erfindungsgemäßen Verfahrens wird Benzol mit Ethylen mit Molverhältnis Benzol : Ethylen von 1,5 bis 3,4 umgesetzt. Als Katalysatoren werden die üblichen Friedel-Crafts-Katalysatoren, vorzugsweise Aluminiumchlorid, verwendet. Der Katalysator wird in Form einer Lösung in üblicher Weise in die Reaktionszone eingeführt. Als Lösungsmittel hierfür können Nebenprodukte der Reaktion, insbesondere Diethylbenzol, verwendet werden. Die bei der Synthese verbrauchte Menge an Katalysator wird ergänzt im Verhältnis von 0,001 – 0,0005 Mol Aluminiumchlorid, gerechnet als trockenes $AlCl_3$, und bezogen auf 1 Mol Benzol. In der Regel werden zusätzlich Cokatalysatoren auf Basis von wasserfreien Halogenwasserstoffen, bevorzugt Chlorwasserstoff, in üblichen und dem Fachmann bekannten Mengen mitverwendet. Die Umsetzung erfolgt bei Temperaturen von 100 bis 160° C, vorzugsweise zwischen 120 und 150° C. Die Drucke können im Bereich von 0,005 bis 5 bar liegen. Die Verweilzeiten in der Reaktionszone können von 5 bis 60 Minuten, insbesondere von 10 bis 40 Minuten betragen. Das erfindungsgemäße Verfahren wird in vorwiegend einer Reaktionszone durchgeführt. Werden mehrere Reaktionszonen angewendet, so geschieht dies vorzugsweise in hintereinander geschaltetem Betrieb. Der Auslauf aus der Reaktionszone wird in üblicher Weise in einem liegenden Behälter nach entsprechender Abkühlung in eine spezifisch leichtere organische Phase a) und eine überwiegend die komplexen organischen Aluminiumchloridverbindungen enthaltende schwerere organische Phase b) aufgetrennt. Die spezifisch schwerere organische Phase b) wird in die Reaktionszone zurückgeführt. Die spezifisch leichtere organische Phase a) wird vom Fachmann oft als Rohethylbenzolgemisch bezeichnet und enthält im wesentlichen das Wertprodukt, ferner nichtumgesetztes Benzol und höhere Ethylbenzole sowie Rückstände zum Teil unbekannter Natur. Die Aufarbeitung dieser Phase erfolgt durch fraktionierte Destillation unter Gewinnung des Wertproduktes. Die bei der fraktionierten Destillation in den verschiedenen Kolonnen, der Benzolkolonne, bzw. Polyethylbenzolkolonne anfallenden Produkte werden in die Reaktionszone zurückgeführt. Auch der Rückstand der Polyethylbenzolkolonne kann gegebenenfalls teilweise zurückgeführt werden (vgl. Einzelheiten P 3 009 932.3 – O. Z. 34 347). Für die Entfernung von Aluminiumchloridresten in der spezifisch leichteren organischen Phase werden übliche Verfahren angewendet, z. B. Wäsche mit Natronlauge und dergleichen.

Die spezifisch schwerere organische Phase, die im wesentlichen die komplexen organischen Aluminiumchloridverbindungen enthält, wird in den liegenden Behälter abgezogen und in den Reaktor zurückgepumpt. Für die Wiederverwendung der spezifisch schwereren organischen Phase bei Reaktionstemperaturen von 130–160°C in der Reaktionszone ist entscheidend, daß wasserfreies Benzol eingesetzt wird und daß die Abscheidung der spezifisch schwereren organischen Phase in dem erwähnten liegenden Behälter bei Temperaturen unterhalb von 40°C erfolgt. Sofern diese Voraussetzungen erfüllt sind, wird die katalytische Aktivität der in den Kreislauf zurückgeführten komplexen organischen Aluminiumchloridverbindungen bei Reaktionstemperaturen auch oberhalb von 130°C nicht beeinträchtigt. Wesentlich für die Durchführung des erfindungsgemäßen Verfahrens ist, daß der Auslauf aus der Reaktionszone, bzw. der letzten Reaktionszone vor der Phasentrennung in ein Druckgefäß eingeführt wird und in diesem unter intensiver Durchmischung behandelt wird. Der für die Aufnahme des Reaktionsgemisches verwendete Druckreaktor kann in beliebiger Weise gestaltet sein und übliche für die Durchmischung des Reaktionsgemisches erforderliche Vorrichtungen enthalten. Beispielsweise kann er in Form eines Rührreaktors mit intensiver radialer Durchmischung ausgebildet sein. Die Durchmischung kann auch dadurch erfolgen, daß in dem der Reaktionszone nachgeschalteten Druckreaktor eine Entnahme stattfindet und ein Teil des Inhaltes durch Umpumpen wieder in diesen Druckreaktor zurückgeführt wird. Es muß auf jeden Fall für eine intensive Durchmischung des Reaktionsgemisches, d. h. für eine ausreichende rasche Gleichgewichtseinstellung, gesorgt werden. Die Innenauskleidung dieses Druckreaktors sollte, wie die der eigentlichen Reaktionszone, gegen die verwendeten Lewis-Säuren stabil sein. Die Behandlung in dem Druckgefäß kann bei Temperaturen von 40°C und bis zu 160°C durchgeführt werden. Aus energetischen und kinetischen Gründen wird aber eine Temperatur gewählt, die lediglich wenig unterhalb der Temperatur der Reaktionszone liegt, d. h. im wesentlichen werden Temperaturen im Bereich zwischen 120 und 150°C eingestellt. Unter den vorstehend erwähnten Bedingungen verläuft die Gleichgewichtseinstellung relativ schnell und katalysatorschonend. Die Verweilzeit des Auslaufes in dem Druckgefäß soll mindestens 20 Minuten und kann bis zu 40 Minuten betragen. Der Fachmann ist in der Wahl der Gestaltung dieses Druckgefäßes (Rührgefäß) weitgehend frei; bei niedrigen Temperaturen verlangsamt sich die Gleichgewichtseinstellung, und es müssen daher Behälter mit größerem Volumen verwendet werden. In dem genannten Temperaturbereich von 120–150°C stellt sich in Abhängigkeit von der gewählten Temperatur unterschiedlich schnell ein thermodynamisches Gleichgewicht ein, wobei sich die Zusammensetzung des Rohethylbenzols zugunsten einer Zunahme des Ethylbenzolgehaltes in diesem Rohethylbenzol verändert. Nach den uns vorliegenden Ergebnissen stellt sich ungefähr folgendes Gleichgewicht in dem Druckgefäß ein:

| Art des Stoffes | Gehalt in Gew.-% |
| --- | --- |
| Unbekannte | 1,5 |
| Benzol | 39,0 |
| Ethylbenzol | 46,0 |
| Polyethylbenzol | 13,5 |

Es wird angenommen, daß die Gleichgewichtseinstellung durch Austausch von Aromaten aus dem organischen Aluminiumchloridkomplex und dem Rohethylbenzol erfolgt. Nach Beendigung im Druckgefäß entspricht demnach die Ligandenzusammensetzung des Katalysators der des Rohethylbenzols.

Das erfindungsgemäße Verfahren kann gegenüber dem bisherigen Verfahren zur Durchführung der Synthese von Ethylbenzol in heterogener Phase auch bei Temperaturen oberhalb 130°C durchgeführt werden. Wenn auch dabei die Konzentration an Polyethylbenzolen in der Reaktionszone zunimmt, stellt sich durch die erfindungsgemäße Nachbehandlung im Druckgefäß die vorstehend genannte höhere Konzentration an Ethylbenzol ein. Das erfindungsgemäße Verfahren ist auch flexibler als es Verfahren vom Stand der Technik sind, da auch weniger als 2 Mol Benzol pro Mol Ethylen angewendet werden können, ohne daß die Ethylbenzolausbeute sich verringert. Auch der Aluminiumchloridversuch ist beim erfindungsgemäßen Verfahren niedrig, er beträgt zwischen 0,1 bis 0,15 Molprozent pro Mol Ethylbenzol. Die Überführung des Inhalts der Reaktionszone in das Druckgefäß kann gegebenenfalls unter Druckabsenkung vorgenommen werden. Es ist auch möglich, in das Druckgefäß Cokatalysator zuzuführen. Falls von der Maßnahme der Druckabsenkung Gebrauch gemacht wird, so wird im Druckgefäß ein Druck eingestellt, der 0,5 bis 2 bar unterhalb des in der Reaktionszone angewendeten Druckes liegt.

Die Erfindung wird nachstehend anhand der Beispiele näher erläutert. Die in den Beispielen genannten Prozente beziehen sich, wenn nichts anderes vermerkt ist, auf das Gewicht.

Beispiel 1

Die Versuche zur Ermittlung der Gleichgewichtseinstellung wurden zunächst in einer Laborapparatur durchgeführt. Aus einem technischen Reaktor, in dem die Ethylbenzolsynthese in einer Reaktionszone kontinuierlich in heterogener Phase bei einer Temperatur von 135°C durchgeführt wurde, wurden 2000 g abgezogen und in einer Glasapparatur intensiv gerührt. Es wurden Temperaturen im Bereich von 20 bis 150°C gewählt und bei verschiedenen Temperaturen in diesem Intervall die Gleichgewichtszusammensetzung ermittelt. Zur Aufrechterhaltung der eingestellten Temperatur befand sich die Glasapparatur in einem isothermen Bad. Die Probenahme erfolgte in Abständen von 20 bzw. 30 Minuten. Nach der Trennung der spezifisch leichteren (Rohethylbenzolphase) von der spezifisch schwereren Phase wurde das Rohethylbenzol gaschromatographisch analysiert. Die erhaltenen Analysenwerte sind, bezogen auf 100% der Phase, in der Tabelle aufgeführt.

Beispiel 2

Wie vorstehend beschrieben, wurde auch in einer technischen Anlage die Gleichgewichtseinstellungen ermittelt. Zu diesem Zweck wurde als Druckreaktor die Reaktionszone verwendet. Dazu mußte lediglich die Ethylen-, Benzol- und Polyethylbenzol-Zufuhr in die Reaktionszone abgestellt werden. Die Versuche wurden bei 135°C durchgeführt. Der Reaktorinhalt wurde umgepumpt und danach der zeitliche Verlauf der Änderung der Zusammensetzung des Rohethylbenzols bestimmt. Die Ergebnisse sind in der Tabelle aufgeführt. Die vergleichende Betrachtung der in der Tabelle aufgeführten Versuchsergebnisse zeigt, daß zwischen den Ergebnissen im Labor und im technischen Versuch keine Unterschiede bestehen und daß, wie bereits dargelegt, nach bestimmten Verweilzeiten bei höheren Temperaturen sich ein Gleichgewicht einstellt, bei dem Ethylbenzolwerte im Bereich von ca. 44—45,9% im Rohethylbenzol gefunden werden.

## Tabelle

|  | Temperatur (°C) | Mischzeit in Minuten | Benzol (%) | Ethylbenzol (%) | Polyethyl-benzol (%) | Unbekannte (%) |
|---|---|---|---|---|---|---|
| Labor | 20 | 0 | 42,59 | 40,50 | 15,12 | 1,79 |
|  |  | 30 | 42,34 | 41,67 | 14,18 | 1,81 |
|  |  | 60 | 41,82 | 41,63 | 14,16 | 1,94 |
|  |  | 90 | 42,50 | 41,59 | 14,17 | 1,74 |
|  |  | 120 | 41,78 | 42,06 | 14,42 | 1,74 |
|  |  | 150 | 42,80 | 41,88 | 13,53 | 1,79 |
|  |  | 1 110 | 41,20 | 43,87 | 13,57 | 1,56 |
|  | 80 | 1 170 | 39,88 | 44,88 | 13,31 | 1,93 |

Fortsetzung

| | Temperatur (°C) | Mischzeit in Minuten | Benzol (%) | Ethylbenzol (%) | Polyethyl-benzol (%) | Unbekannte (%) |
|---|---|---|---|---|---|---|
| | 60 | 0 | 42,79 | 40,24 | 15,03 | 1,94 |
| | | 30 | 41,50 | 41,10 | 15,59 | 1,81 |
| | | 60 | 41,53 | 41,78 | 15,00 | 1,69 |
| | | 90 | 40,99 | 42,67 | 14,55 | 1,79 |
| | | 120 | 40,91 | 43,12 | 14,16 | 1,81 |
| | | 150 | 40,90 | 43,79 | 13,43 | 1,88 |
| | | 180 | 40,76 | 43,69 | 13,67 | 1,88 |
| | 100 | 0 | 42,01 | 40,90 | 15,24 | 1,85 |
| | | 30 | 41,65 | 42,02 | 14,61 | 1,72 |
| | | 60 | 40,74 | 42,78 | 14,74 | 1,74 |
| | | 90 | 40,29 | 43,48 | 14,32 | 1,91 |
| | | 120 | 40,24 | 44,57 | 13,40 | 1,79 |
| | 125 | 0 | 44,72 | 40,95 | 12,67 | 1,66 |
| | | 30 | 40,19 | 44,87 | 13,10 | 1,84 |
| | | 60 | 39,24 | 45,86 | 13,15 | 1,74 |
| | 150 | 0 | 42,78 | 41,84 | 13,59 | 1,79 |
| | | 20 | 39,57 | 45,12 | 13,77 | 1,54 |
| | | 40 | 37,83 | 45,31 | 15,35 | 1,61 |
| Betrieb | 135 | 0 | 44,26 | 41,20 | 12,74 | 1,80 |
| | | 30 | 40,76 | 44,83 | 12,69 | 1,72 |
| | | 60 | 40,34 | 44,64 | 13,28 | 1,74 |

**Patentansprüche**

1. Verfahren zur kontinuierlichen Herstellung von Ethylbenzol in heterogener Phase durch Alkylieren von Benzol mit Ethylen im Molverhältnis Benzol/Ethylen im Bereich von 1,5 – 3,4 in Gegenwart von Katalysatoren auf Basis von komplexen organischen Aluminiumchloridverbindungen in Mengen von 0,06 – 0,02 Mol $AlCl_3$, bezogen auf 1 Mol Benzol, und gegebenenfalls von Cokatalysatoren in üblichen Mengen auf Basis von wasserfreiem Halogenwasserstoff

bei Temperaturen von 130 bis 160° C
und
Drücken von 2 bis 5 bar
und
Verweilzeiten von 5 bis 60 Minuten
in einer oder gegebenenfalls mehreren Reaktionszone(n), Auftrennung des Auslaufs aus der(n) Reaktionszone(n) in eine

a) spezifisch leichtere organische Phase, die das Wertprodukt, nichtumgesetztes Benzol und höhere Ethylbenzole und Rückstände enthält,
und eine
b) spezifisch schwerere organische Phase, die die komplexen organischen Aluminiumchloridverbindungen enthält,

Aufarbeitung der spezifisch leichteren Phase a) durch Destillation unter Abtrennung des Wertproduktes und unter Rückführung von nichtumgesetztem Benzol, und Rückführung der spezifisch schwereren Phase b) in die Reaktionszone
und Ergänzung von Verlusten an Katalysator und/oder Cokatalysator, dadurch gekennzeichnet, daß man den Auslauf aus der(n) Reaktionszone(n) vor der Phasentrennung in ein Druckgefäß einführt und in diesem mindestens 20 Minuten bis 60 Minuten bei Temperaturen von 120 – 160° C intensiv mischt (behandelt).
2. Verfahren nach Anspruch 1, dadurch gekennzeichnet, daß die Behandlung bei niedrigeren Drucken, als im Reaktionsgefäß und gegebenenfalls unter Zuführung von Cokatalysator durchgeführt wird.

## Claims

1. A Process for the continuous preparation of ethylbenzene in a heterogeneous-phase reaction, by alkylating benzene with ethylene, using a molar ratio of benzene to ethylene of from 1.5 to 3.4 : 1, in the presence of a catalyst based on a complex organic aluminium chloride compound and used in an amount of from 0.06 to 0.02 mole of $AlCl_3$ per mole of benzene, and in the presence or absence of a conventional amount of an anhydrous hydrogen halide as co-catalyst, at from 130 to 160° C and under a pressure of from 2 to 5 bar, using a residence time of from 5 to 60 minutes and carrying out the reaction in at least one zone, the material discharged from the zone being separated into a) a less dense organic phase which contains the useful product, unconverted benzene, higher ethylbenzenes and residues, and b) a denser organic phase which contains the complex organic aluminium chloride compound, and the less dense phase a) being worked up by distillation, with isolation of the useful product and recycling of unconverted benzene, whilst the denser phase b) is recycled to the reaction zone and losses of catalyst and/or co-catalyst are made up, in which process the material leaving the reaction zone is introduced into a pressure vessel before phase separation and it is vigorously mixed (treated) therein for not less than 20 minutes and up to 60 minutes at from 120 to 160° C.
2. A process as claimed in claim 1, wherein the treatment is carried out at a lower pressure than in the reaction vessel, and with or without the addition of co-catalyst.

## Revendications

1. Procédé pour la préparation en continu de l'éthyl-benzène
en phase hétérogène
par alcoylation du benzène par l'éthylène en un rapport molaire benzène-éthylène dans la gamme de 1,5 à 3,4
en présence de catalyseurs à base de composés organiques complexes du chlorure d'aluminium en proportions de 0,06 à 0,02 mole de $AlCl_3$ par mole de benzène
et éventuellement
de co-catalyseurs à base d'hydrogène halogéné anhydre en des proportions usuelles
à des températures de 130 à 160° C,
sous des pressions de 2 à 5 bars et
avec des durées de séjour de 5 à 60 minutes
dans une zone de réaction ou éventuellement dans plusieurs zones de réaction,
séparation de l'effluent de la ou des zones de réaction en

a) une phase organique spécifiquement plus légère, contenant le produit recherché, du benzène non transformé, des éthyl-benzènes supérieurs et des résidus,
et

b) une phase organique spécifiquement plus lourde, contenant les composés organiques complexes du chlorure d'aluminium,

traitement de la phase spécifiquement plus légère a) par distillation avec séparation du produit recherché et recyclage du benzène non transformé, ainsi que recyclage de la phase specifiquement plus lourde b) dans la zone réactionnelle avec compensation des pertes en catalyseur et (ou) co-catalyseur, caractérisé en ce que, préablement à la séparation des phases, l'effluent de la ou des zones de réaction est introduit dans un réservoir résistant à la pression, dans lequel il est soumis pendant au moins 20 minutes et jusqu'à 60 minutes, à des températures de 120 à 160°C, à un malaxage (traitement) intense.

2. Procédé suivant la revendication 1, caractérisé en ce que le traitement est effectué à des pressions inférieures à celles régnant dans le réacteur avec addition éventuelle de co-catalyseur.